# NEW EUROPEAN PATENT SPECIFICATION

(11) **EP 0 891 420 B2**
(45) Date of publication and mention of the opposition decision: **20.11.2013**
(45) Mention of the grant of the patent: 02.02.2005
(21) Application number: 97915628.8
(22) Date of filing: 01.04.1997
(51) Int. Cl.: C12N 7/00, A61K 39/145

(54) **PROCESSES FOR THE REPLICATION OF INFLUENZA VIRUSES IN CELL CULTURE, AND THE INFLUENZA VIRUSES OBTAINABLE BY THE PROCESS**
VERFAHREN FUER DIE REPLIKATION VON INFLUENZA IN ZELLKULTUR UND DIE BEI DIESEM VERFAHREN HERGESTELLTEN INFLUENZA-VIREN
PROCEDES DE REPLICATION DES VIRUS DE LA GRIPPE DANS UNE CULTURE CELLULAIRE, ET VIRUS DE LA GRIPPE OBTENUS SELON CE PROCEDE

(30) Priority: 01.04.1996 DE 19612967
(43) Date of publication of application: 20.01.1999
(60) Divisional application: 05075182.5
(73) Proprietor: Novartis Vaccines and Diagnostics GmbH, 35041 Marburg (DE)
(72) Inventor: GRÖNER, Albrecht, D-64342 Seeheim (DE)
(74) Representative: Hallybone, Huw George
(86) International application number: PCT/IB1997/000404
(87) International publication number: WO 1997/037001

(56) References cited:
- GB-A- 1 070 764
- US-A- 4 500 513
- ADVANCES IN EXPERIMENTAL MEDICINE AND BIOLOGY, vol. 397, 1996, pages 141-151, XP002039317 O.-W. MERTEN ET AL.: "PRODUCTION OF INFLUENZA VIRUS IN CELL CULTURES FOR VACCINE PREPARATION"
- REVISTA DE FARMÀCIA E BIOQU MICA DA UNIVERSIDADE DE SÃO PAULO, vol. 29, no. 2, July 1993 - December 1993, pages 89-95, XP002039318 DALVA A. PORTARI MANCINI ET AL.: "AVALIACÃO DA TRIPSINA NA MULTIPLICACÃO DE VÍRUS INFLUENZA EM CULTURAS DE CÉLULAS MDCK"

## Description

The present invention relates to processes for the replication of influenza viruses in cell culture at reduced temperatures, and to the influenza viruses obtainable by the process described and to vaccines which contain viruses of this type or constituents thereof.

All influenza vaccines which have been used since the 40s until today as permitted vaccines for the treatment of humans and animals consist of one or more virus strains which have been replicated in embryonate hens' eggs. These viruses are isolated from the allantoic fluid of infected hens' eggs and their antigens are used as vaccine either as intact virus particles or as virus particles disintegrated by detergents and/or solvents - so-called cleaved vaccine - or as isolated, defined virus proteins - so-called subunit vaccine. In all permitted vaccines, the viruses are inactivated by processes known to the person skilled in the art. Even the replication of live attenuated viruses, which are tested in experimental vaccines, is carried out in embryonate hens' eggs.
The use of embryonate hens' eggs for vaccine production is time-, labor- and cost-intensive. The eggs - from healthy flocks of hens monitored by veterinarians - have to be incubated before infection, customarily for 12 days. Before infection, the eggs have to be selected with respect to living embryos, as only these eggs are suitable for virus replication. After infection the eggs are again incubated, customarily for 2 to 3 days. The embryos still alive at this time are killed by cold and the allantoic fluid is then obtained from the individual eggs by aspiration. By means of laborious purification processes, substances from the hen's egg which lead to undesired side effects of the vaccine are separated from the viruses, and the viruses are concentrated. As eggs are not sterile (pathogen-free), it is additionally necessary to remove and/or to inactivate pyrogens and all pathogens which are possibly present. To increase the virus yield, the replication of the influenza viruses in hens' eggs as a rule is carried out at reduced temperatures (about 34°C). Even viruses which cause respiratory diseases can be replicated in cell culture. Here too, in some cases reduced temperatures are used (about 33°C), which, however, have no effect on the quality of a vaccine which may be obtained, but only favor replication.
Viruses of other vaccines such as, for example, rabies viruses, mumps, measles and rubella viruses, polio viruses and FSME viruses can be replicated in cell cultures. As cell cultures originating from tested cell banks are pathogen-free and, in contrast to hens' eggs, are a defined virus replication system which (theoretically) is available in almost unlimited amounts, they make possible economical virus replication under certain circumstances even in the case of influenza viruses. Economical vaccine production is possibly also achieved in that virus isolation and purification from a defined, sterile cell culture medium appears simpler than from the strongly protein-containing allantoic fluid.
The isolation and replication of influenza viruses in eggs leads to a selection of certain phenotypes, of which the majority differ from the clinical isolate. In contrast to this is the isolation and replication of the viruses in cell culture, in which no passage-dependent selection occurs (Oxford, J.S. et al., J. Gen. Virology 72 (1991), 185 - 189; Robertson, J.S. et al., J. Gen. Virology 74 (1993) 2047 - 2051). For an effective vaccine, therefore, virus replication in cell culture is also to be preferred from this aspect to that in eggs. It is known that influenza viruses can be replicated in cell cultures. Beside hens' embryo cells and hamster cells (BHK21-F and HKCC), MDBK cells, and in particular MDCK cells have been described as suitable cells for the in-vitro replication of influenza viruses (Kilbourne, E. D., in: Influenza, pages 89 - 110, Plenum Medical Book Company - New York and London; 1987). A prerequisite for a successful infection is the addition of proteases to the infection medium, preferably trypsin or similar serine proteases, as these proteases extracellularly cleave the precursor protein of hemagglutinin [HAₒ] into active hemagglutinin [HA₁ and HA₂]. Only cleaved hemagglutinin leads to the adsorption of the influenza viruses on cells with subsequent virus assimilation into the cell (Tobita, K. et al., Med. Microbiol. Immunol., 162 (1975), 9 - 14; Lazarowitz, S.G. & Choppin, P.W., Virology, 68 (1975) 440 - 454; Klenk, H.-D. et al., Virology 68 (1975) 426 - 439) and thus to a further replication cycle of the virus in the cell culture.
The Patent US 4 500 513 described the replication of influenza viruses in cell cultures of adherently growing cells. After cell proliferation, the nutrient medium is removed and fresh nutrient medium is added to the cells with infection of the cells with influenza viruses taking place simultaneously or shortly thereafter. A given time after the infection, protease (e.g. trypsin) is added in order to obtain an optimum virus replication. The viruses are harvested, purified and processed to give inactivated or attenuated vaccine. Economical influenza-virus replication as a prerequisite for vaccine production cannot be accomplished, however, using the methodology described in the patent mentioned, as the change of media, the subsequent infection as well as the addition of trypsin which is carried out later necessitate opening the individual cell-culture vessels several times and is thus very labor-intensive. Furthermore, the danger increases of contamination of the cell culture by undesirable microorganisms and viruses with each manipulation of the culture vessels. A more cost-effective alternative is cell proliferation in fermenter systems known to the person skilled in the art, the cells growing adherently on microcarriers. The serum necessary for the growth of the cells on the microcarriers (customarily fetal calf serum), however, contains trypsin inhibitors, so that even in this production method a change of medium to serum-free medium is necessary in order to achieve the cleavage of the influenza hemagglutinin by trypsin and thus an adequately high virus replication. Thus this methodology also requires opening of the culture vessels several times and thus brings with it the increased danger of contamination. In the patent US 4500513, viral replication is performed at 34-37°C. Similarly, Mancini & Yano [Rev Farm Bioquim Univ S.Paulo (1993) 29:89-95] describe an influenza cell culture process where virus is replicated at 35°C. These authors used MDCK cells grown in Eagle medium supplemented with fetal bovine serum and neomycin. Trypsin was added to the cultured cells after infection with the influenza virus.

The present invention is thus based on the object of making available processes which make possible simple and economical influenza virus replication in cell culture and lead to a highly efficacious vaccine.

This object is achieved by the provision of the embodiments indicated in the patent claims.

The invention thus relates to a process for making a vaccine in cell culture, as defined in the claims in which MDCK 33016 cells which can be infected by influenza viruses are cultured in cell culture in suspension in serum-free medium, the cells are infected with influenza viruses and after infection are cultured at a temperature in the range from 30 to 36°C for virus replication. Before or during the infection by influenza virus, a protease is added to the cell culture. Further details of the protease treatment are given below.

In a preferred embodiment of the process according to the invention, the culturing of the infected cells for virus replication is carried out at 32 to 34°C and particularly preferably at 33°C.

It has surprisingly been found that by the replication of the influenza viruses in infected cells at reduced temperatures, viruses are obtained which have an appreciably higher efficacy as vaccine than those viruses which are obtained by replication at 37°C. Replication at 37°C, the customarily used temperature for influenza replication in cell culture, admittedly leads to comparatively high virus yields in a short time. However, the viruses thus produced have a low efficacy as vaccine in comparison with viruses which are prepared by the process according to the invention.

In the context of the present invention, a cell line was established from the kidney cell line MDCK, which cell line is adapted to growth in suspension in serum-free medium and thereby makes possible particularly simple and efficient culturing and virus replication. This cell line, MDCK 33016, is used in the process according to the invention. It was deposited under the deposit number DSM ACC 2219 on June 7, 1995 according to the requirements of the Budapest Convention on the Recognition of the Deposition of Microorganisms for the purposes of patenting in the German Collection of Microorganisms (DSM) in Brunswick (Federal Republic of Germany), which is recognized as the international deposition site.

For culturing the cells in the process according to the invention, the customary methods known to the person skilled in the art can be used for cell culture, in particular those which are already known for the replication of influenza viruses in cell culture. The carrying-out of the process according to the invention using cells which grow in suspension, in serum-free medium, makes possible particularly simple and efficient virus replication. Culturing of the cells in suspension can in this case be carried out both in the batch process and in the perfusion system, e.g. in a stirred vessel fermenter, using the cell retention systems known to the person skilled in the art, such as, for example, centrifugation, filtration, spin filters and the like.

The culturing of the cells is carried out as a rule at a regulated pH which is preferably in the range from pH 6.6 to pH 7.8, in particular in the range from pH 6.8 to pH 7.3.
Furthermore, the pO₂ value can advantageously be regulated and is then as a rule between 25% and 95%, in particular between 35% and 60% (based on the air saturation).
The infection of the cells cultured in suspension is preferably carried out when the cells in the batch process have reached a cell density of about 8 to 25 x 10⁵ cells/ml or about 5 to 20 × 10⁶ cells/ml in the perfusion system.
The infection of the cells with influenza viruses is preferably carried out at an m.o.i. (multiplicity of infection) of about 0.0001 to 10, preferably of 0.002 to 0.5.

The addition of a protease which brings about the cleavage of the precursor protein of hemagglutinin [HAₒ] and thus the adsorption of the viruses to the cells, can be carried out according to the invention shortly before or simultaneously with the infection of the cells with influenza viruses. If the addition is carried out simultaneously with the infection, the protease can either be added directly to the cell culture to be infected or, for example, as a concentrate together with the virus inoculate. The protease is preferably a serine protease, and particularly preferably trypsin.

If trypsin is used, the final concentration added in the culture medium is advantageously 1 to 200 *µ*g/ml, preferably 5 to 50 *µ*g/ml, and particularly preferably 5 to 30 *µ*g/ml.
After infection, the infected cell culture is cultured further to replicate the viruses, in particular until a maximum cytopathic effect or a maximum amount of virus antigen can be detected.

In a preferred embodiment of the process, the harvesting and isolation of the replicated influenza viruses is carried out 2 to 10 days, preferably 3 to 7 days, after infection. To do this, for example, the cells or cell residues are separated from the culture medium by means of methods known to the person skilled in the art, for example by separators or filters. Following this the concentration of the influenza viruses present in the culture medium is carried out by methods known to the person skilled in the art, such as, for example, gradient centrifugation, filtration, precipitation and the like.

The influenza viruses are formulated by known methods to give a vaccine for administration to humans or animals. As already explained above, influenza viruses of this type have a higher efficacy as vaccine than influenza viruses which are obtained by replication at 37°C in cell culture.
The immunogenicity or efficacy of the influenza viruses obtained as vaccine can be determined by methods known to the person skilled in the art, e.g. by means of the protection imparted in the exposure experiment or as antibody titers of virus-neutralizing antibodies.
The determination of the amount of virus or antigen produced can be carried out, for example, by the determination of the amount of hemagglutinin by methods known to the person skilled in the art. It is known, for example, that cleaved hemagglutinin binds to erythrocytes of various species, e.g. to hens' erythrocytes. This makes possible a simple and rapid quantification of the viruses produced or of the antigen formed by appropriate detection methods.

By means of comparison experiments in animal models, it was demonstrated that influenza viruses according to the invention produce an appreciably higher titer of neutralizing antibodies than viruses replicated at 37°C and thereby impart an appreciably better protection against influenza virus infection. In experiments with mice as an animal model, the titer of neutralizing antibodies was, for example, higher by at least a factor of 42 weeks after vaccination than the titer of neutralizing antibodies after inoculation with influenza viruses which had been replicated at 37°C. 4 weeks after the inoculation, the titer of neutralizing antibodies was higher by at least a factor of 17 and in some cases up to 27 times higher. If a revaccination was carried out, the titer of neutralizing antibodies could be higher by a factor of over 60 when using influenza viruses according to the invention in comparison with influenza viruses which had been replicated at 37°C. Accordingly, the survival rate of animals in an exposure experiment using an administration of 1000 LD₅₀ (lethal dose 50%) can be increased from 1/10 to at least 8/10, preferably to 9/10 and particularly preferably to 10/10 (100%).

The vaccines obtained from the process according to the invention can optionally contain the additives customary for vaccines, in particular substances which increase the immune response, i.e. so-called adjuvants, e.g. hydroxides of various metals, constituents of bacterial cell walls, oils or saponins, and moreover customary pharmaceutically tolerable excipients.

The viruses can be present in the vaccines as intact virus particles, in particular as live attenuated viruses. For this purpose, virus concentrates are adjusted to the desired titer and either lyophilized or stabilized in liquid form.

In a further preferred embodiment, the vaccines can contain disintegrated, i.e. inactivated, or intact, but inactivated viruses. For this purpose, the infectiousness of the viruses is destroyed by means of chemical and/or physical methods (e.g. by detergents or formaldehyde). The vaccine is then adjusted to the desired amount of antigen and after possible admixture of adjuvants or after possible vaccine formulation, dispensed, for example, as liposomes, microspheres or slow release formulations.

In a further preferred embodiment, the vaccine can finally be present as subunit vaccine, i.e. it can contain defined, isolated virus constituents, preferably isolated proteins of the influenza virus. These constituents can be isolated from the influenza viruses by methods known to the person skilled in the art.

The examples illustrate the invention.

### Example 1 (Reference example)

### Replication of influenza viruses in MDCKcells at 33°C

MDCK cells (ATCC CCL 34) were replicated in cell culture bottles (Eagle's MEM [EMEM] using 2% FCS, incubation at 37°C for 4 days). The resulting dense cell lawn was detached from the vessel wall using trypsin solution, the cells were isolated and the cell concentrate was resuspended in serum-containing medium. The cells were inoculated into roller bottles (200 ml/bottle) at a cell density of 5 × 10⁵ cells/ml and incubated at 37°C at 4 rpm. After 2 days, the cells were infected with influenza viruses. To do this, the medium above the dense cell lawn was removed and replaced by serum-free EMEM. Influenza virus A/PR/8/34 with an m.o.i. (multiplicity of infection) of 0.1 and trypsin in a final concentration of 25 *µ*g/ml were added to the medium. Two roller bottles in each case were incubated at 37°C or at 33°C. The virus replication was determined as amount of antigen (measured as hemagglutinin units) and as infectiousness (measured in the CC ID₅₀ test) was determined and is shown in Table 1.

**Table 1**

| **Replication of influenza A/PR/8/34 in roller bottles (MDCK cell line) after incubation at 37°C and 33°C, measured as antigen content (HA units and infectiousness) (CCID₅₀))** | | | | |
|---|---|---|---|---|
| | HA content | | | CCID₅₀/ml [log₁₀] |
| | 2 dpi | 3 dpi | 4 dpi | 4 dpi |
| 37°C | 1:128 | 1:512 | 1:1024 | 6.4 |
| 33°C | 1:64 | 1:256 | 1:1024 | 5.7 |
| dpi = days after infection | | | | |

The ratios indicated mean that a 1:X dilution of the virus harvest still has hemagglutinating properties. The hemagglutinating properties can be determined, for example, as described in Mayer et al., Virologische Arbeitsmethoden, [Virological Working Methods, Volume 1 (1974), pages 260-261 or in Grist, Diagnostic Methods in Clinical Virology, pages 72-75.

The determination of the CCID₅₀ value can be carried out, for example, according to the method which is described in Paul, Zell- und Gewebekultur [Cell and tissue culture] (1980), p. 395.

### Example 2

### Preparation of a cell line which is adapted to growth in suspension and can be infected by influenza viruses

A cell line which is suited to growth in suspension culture and can be infected by influenza viruses was selected starting from MDCK cells (ATCC CCL34 MDCK (NBL-2), which had been proliferated by means of only a few passages or over several months in the laboratory. This selection was carried out by proliferation of the cells in roller bottles which were rotated at 16 rpm (instead of about 3 rpm as customary for roller bottles having adherently growing cells). After several passages of the cells present suspended in the medium, cell strains growing in suspension were obtained. These cell strains were infected with influenza viruses and the strains were selected which produced the highest virus yield. An increase in the rate of cells growing in suspension during the first passages at 16 rpm is achieved over 1 to 3 passages by the addition of selection systems known to the person skilled in the art, such as hypoxanthine, aminopterin and thymidine, or alanosine and adenine, individually or in combination. The selection of cells growing in suspension is also possible in other agitated cell culture systems known to the person skilled in the art, such as stirred flasks. An example of cells which are adapted to growth in suspension and can be infected by influenza viruses is the cell line MDCK 33016 (DSM ACC2219).

### Example 3

### Replication of influenza viruses in MDCK 33016 cells at 33°C

The cell line MDCK 33016 (DSM ACC2219) growing in suspension was replicated at 37°C in Iscove's medium with a splitting rate of 1:8 to 1:12 twice weekly in a roller bottle which rotated at 16 rpm. 4 days after transfer, a cell count of approximately 7.0 × 10⁵ cells/ml was achieved. Simultaneously with the infection of the now 4-day old cell culture with the influenza strain A/PR/8/34 (m.o.i. = 0.1), the cell culture was treated with trypsin (25 *µ*g/ml final concentration), incubated further at 37°C or 33°C and the virus replication was determined over 3 days (Tab. II).

**Table II**

| **Replication of influenza A/PR/8/34, measured as antigen content (HA units) in roller bottles (MDCK cell line MDCK 33016) after infection of a cell culture without change of medium at an incubation temperature of 37°C or 33°C** | | | |
|---|---|---|---|
| HA content after days after infection (dpi) | | | |
| | 1 dpi | 2 dpi | 3 dpi |
| 37°C | 1:64 | 1:512 | 1:1024 |
| 33°C | 1:16 | 1:128 | 1:1024 |

### Example 4

### Replication of various influenza strains in MDCK 33016 cells (DSM ACC 2219) at 33°C

The cell line MDCK 33016 (DSM ACC 2219) was proliferated at 37°C in Iscove's medium with a splitting rate of 1:8 to 1:12 twice weekly in a roller bottle which rotated at 16 rpm. 4 days after transfer, a cell count of approximately 7.0 × 10⁵ to 10 × 10⁵ cells/ml was achieved. Simultaneously with the infection of the now 4-day old cell culture with various influenza strains (m.o.i. ≈ 0.1), the cell culture was treated with trypsin (25 *µ*g/ml final concentration) and incubated further at 33°C, and the virus replication was determined on the 5th day after infection (Table III).

**Table III**

| **Replication of influenza strains in roller bottles (cell line MDCK 33016) after infection of a cell culture without change of medium, measured as antigen content (HA units)** | |
|---|---|
| Influenza strain | HA content 5 days after infection HA content |
| | |
| A/Singapore | 1:1024 |
| A/Sichuan | 1:256 |
| A/Shanghai | 1:256 |
| A/Guizhou | 1:128 |
| A/Beijing | 1:512 |
| | |
| B/Beijing | 1:256 |
| B/Yamagata | 1:512 |
| | |
| A/PR/8/34 | 1:1024 |
| | |
| A/Equi 1/Prague- | 1:512 |
| A/Equi 2/Miami | 1:256 |
| A/Equi 2 Fontainebleau | 1:128 |
| A/Swine/Ghent | 1:512 |
| A/Swine/Iowa | 1:1024 |
| A/Swine/Arnsberg | 1:512 |

### Example 5

### Preparation of an experimental influenza vaccine

After inoculation in mice, human-pathogenic influenza viruses customarily do not lead to their infection with pathological processes, so that protection experiments with mice are experimentally very difficult to construct. The influenza virus strain A/PR/8/34, however, is adapted to mice and after intranasal administration causes a dose-dependent mortality in mice.

An experimental vaccine was prepared from influenza virus A/PR/8/34 from Example 3 (A/PR/8 replicated at 37°C or 33°C). The influenza viruses in cell culture medium were separated from cells and cell fragments by low-speed centrifugation (2000 g, 20 min, 4°C) and purified by a sucrose gradient centrifugation (10 to 50% (wt/wt) of linear sucrose gradient, 30,000 g, 2 h, 4°C). The influenza virus-containing band was obtained, diluted with PBS pH 7.2 1:10, and sedimented at 20,000 rpm, and the precipitate was taken up in PBS (volume: 50% of the original cell culture medium). The influenza viruses were inactivated with formaldehyde (addition twice of 0.025% of a 35% strength formaldehyde solution at an interval of 24 h, incubation at 20°C with stirring).

10 NMRI mice each, 18 to 20 g in weight, were inoculated with 0.3 ml each of these inactivated experimental vaccines on day 0 and day 28 by subcutaneous injection. 2 and 4 weeks after the inoculation and also 1 and 2 weeks after revaccination, blood was taken from the animals to determine the titer of neutralizing antibodies against A/PR/8/34. To determine the protection rate, the mice were exposed 2 weeks after revaccination (6 weeks after the start of the experiment) by intranasal administration of 1000 LD₅₀ (lethal dose 50%). The results of the experiment are compiled in Table IV.

**Table IV**

| **Efficacy of experimental vaccines: for vaccine A the influenza virus A/PR/8/34 was replicated at 37°C and for vaccine B at 33°C. The titers of neutralizing antibodies against A/PR/8 and also the protection rate after exposure of the mice were investigated.** | | | | | |
|---|---|---|---|---|---|
| Titer of neutralizing antibodies/ml* | | | | | Protection rate Number living/total |
| | 2 w pvacc | 4 w pvacc | 1 w prevacc | 2 w prevacc | |
| 37°C | <28 | 56 | 676 | 1 620 | 1/10 |
| 33°C | 112 | 1 549 | 44 670 | 112 200 | 9/10 |

| | | | | | |
|---|---|---|---|---|---|
| * Weeks after vaccination (w pvacc) and weeks after revaccination (w prevacc) | | | | | |

The experiments confirm that influenza viruses which had been replicated at 37°C in cell culture with a high antigen yield (HA titer) only induced low neutralizing antibody titers in the mouse and barely provided protection, while influenza viruses which had been replicated at 33°C in cell culture also with a high antigen yield (HA titer) induced very high neutralizing antibody titers in the mouse and led to very good protection.

### Example 6 (Reference Example)

### Replication of influenza viruses in MDCK cells at 33°C and efficacy of the vaccine obtained

The cell line MDCK (ATCC CL34) was replicated at 37°C in a cell culture bottle in Eagle's MEM (EMEM) with 2% FCS with a splitting rate of 1:8 to 1:12 twice weekly. 4 days after transformation, a dense cell lawn had resulted. After change of the medium to serum-free EMEM, the cell culture was infected with influenza B/Beijing (m.o.i. = 0.1), trypsin was added to the medium in a final concentration of 25 *µ*g/ml and the infected cell culture bottles were incubated either at 37°C or at 33°C. 4 days after infection, the HA content in both experimental batches was 256 HA units. After low-speed centrifugation to remove cells/cell residues, the viruses in the supernatant were inactivated with formaldehyde (addition two times of 0.025% of a 35% strength formaldehyde solution at an interval of 24 h, incubation at 20°C with stirring). In each experimental section, the adjuvant added was aluminum hydroxide (10% final concentration of a 2% strength Al(OH)₃ solution). Using these experimental vaccines, in each case 3 guinea-pigs (400 to 500 g) per experimental section underwent intraplantar vaccination with 0.2 ml and revaccination 4 weeks afterwards with the same vaccine. To investigate the efficacy of the vaccine, blood samples were taken 2, 4 and 6 weeks after inoculation and tested in the hemagglutination inhibition test and serum neutralization test (cf. Table V).

**Table V**

| **Efficacy of experimental vaccines from influenza B/Beijing after replication of the virus at 37°C and 33°C in cell culture: the serological parameters hemagglutination inhibition and neutralizing antibodies were investigated (average values of 3 guinea-pigs)** | | | | | | |
|---|---|---|---|---|---|---|
| Hemagglutination inhibition Titer | | | | Neutralizing antibodies Titer | | |
| | 2w pvacc* | 4 w pvacc* | 6 w pvacc | 2w pvacc | 4 w pvacc | 6w pvacc |
| 37°C | 85 | 341 | 1024 | 851 | 1290 | 6760 |
| 33°C | 85 | 341 | 853 | 3890 | 22400 | 117490 |

| | | | | | | |
|---|---|---|---|---|---|---|
| * w pvacc = weeks after inoculation (6 w pvacc = 2 weeks after revaccination) | | | | | | |

## Claims

1. A process for making a vaccine for administration to humans or animals comprising:
(a) replication of influenza viruses in cell culture, in which MDCK 33016 cells (DSM ACC 2219) are cultured in cell culture in suspension in serum-free medium, the cells are infected with influenza viruses and after infection are cultured at a temperature in the range from 30°C to 36°C for virus replication, wherein a protease is added to the cultured cells before or during infection with influenza viruses;
(b) formulation of the replicated influenza viruses to give the vaccine.

2. The process as claimed in claim 1, in which the cells are cultured with influenza viruses at a temperature in the range from 32°C to 34°C after infection for virus replication.

3. The process as claimed in claim 2, in which the cells are cultured with influenza viruses at 33°C after infection for virus replication.

4. The process as claimed in any one of claims 1 to 3, in which the protease is a serine protease.

5. The process as claimed in claim 4, wherein the serine protease is trypsin.

6. The process as claimed in any one of claims 1 to 5, in which the harvesting and isolation of the influenza viruses take place 2 to 10 days after infection.

7. The process as claimed in claim 6, in which the harvesting and isolation of the influenza viruses take place 2 to 7 days after infection.

## Patentansprüche

1. Verfahren zur Herstellung eines Impfstoffs zur Verabreichung an Menschen oder Tiere, umfassend
a) Replikation von Influenza-Viren in Zellkultur, wobei MDCK 33016-Zellen (DSM ACC 2219) in Zellkultur in Suspension in serumfreiem Medium gezüchtet werden, die Zellen mit Influenza-Viren infiziert werden und nach Infektion bei einer Temperatur im Bereich von 30°C bis 36°C zur Virusreplikation gezüchtet werden, wobei eine Protease vor oder während der Infektion mit Influenza-Viren zu den gezüchteten Zellen hinzugefügt wird;
b) Formulierung der replizierten Influenza-Viren, um den Impfstoff zu erhalten.

2. Verfahren nach Anspruch 1, in dem die Zellen mit Influenza-Viren bei einer Temperatur im Bereich von 32°C bis 34°C nach Infektion zur Virusreplikation gezüchtet werden.

3. Verfahren nach Anspruch 2, in dem die Zellen mit Influenza-Viren bei 33°C nach Infektion zur Virusreplikation gezüchtet werden.

4. Verfahren nach einem der Ansprüche 1 bis 3, in dem die Protease eine Serin-Protease ist.

5. Verfahren nach Anspruch 4, wobei die Serin-Protease Trypsin ist.

6. Verfahren nach einem der Ansprüche 1 bis 5, in dem das Ernten und Gewinnen der Influenza-Viren 2 bis 10 Tage nach der Infektion stattfindet.

7. Verfahren nach Anspruch 6, in dem das Ernten und Gewinnen der Influenza-Viren 2 bis 7 Tage nach der Infektion stattfinden.

## Revendications

1. Procédé de préparation d'un vaccin destiné à être administré à l'homme ou à l'animal comprenant :
(a) la réplication de virus de la grippe en culture cellulaire, dans laquelle des cellules MDCK 33016 (DSM ACC 2219) sont mises en culture dans des cultures cellulaires en suspension dans du milieu dépourvu de sérum, les cellules sont infectées avec des virus de la grippe et, après l'infection, sont mises en culture à une température comprise dans le domaine de 30°C à 36°C pour la réplication virale, une protéase étant ajoutée aux cellules en culture avant ou pendant l'infection avec les virus de la grippe ;
(b) la formulation des virus de la grippe répliqués pour donner le vaccin.

2. Procédé selon la revendication 1, dans lequel les cellules sont mises en culture avec des virus de la grippe à une température comprise dans le domaine de 32°C à 34°C après infection, pour la réplication virale.

3. Procédé selon la revendication 2, dans lequel les cellules sont mises en culture avec des virus de la grippe à 33°C après infection, pour la réplication virale.

4. Procédé selon l'une quelconque des revendications 1 à 3, dans lequel la protéase est une protéase à sérine.

5. Procédé selon la revendication 4, dans lequel la protéase à sérine est la trypsine.

6. Procédé selon l'une quelconque des revendications 1 à 5, dans lequel la récupération et l'isolement des virus de la grippe ont lieu 2 à 10 jours après l'infection.

7. Procédé selon la revendication 6, dans lequel la récupération et l'isolement des virus de la grippe ont lieu 2 à 7 jours après l'infection.
